# EUROPEAN PATENT SPECIFICATION

(11) **EP 3 308 829 B1**
(45) Date of publication and mention of the grant of the patent: **29.04.2020**
(21) Application number: 17190216.6
(22) Date of filing: 08.09.2017
(51) Int. Cl.: A61N 1/20, A61F 2/02, A61L 27/24, A61L 27/36, A61L 27/50, A61L 27/56, A61L 27/58, A61B 17/11, A61F 2/28, A61F 2/04, A61L 27/00, A61B 17/00, A61F 2/30, A61N 1/05

(54) **ELECTROSTIMULATIVE GRAFT PRODUCTS**
ELEKTROSTIMULATIVE TRANSPLANTATPRODUKTE
PRODUITS DE GREFFE ÉLECTROSTIMULATIVE

(30) Priority: 10.09.2016 US 201662393001 P
(43) Date of publication of application: 18.04.2018
(73) Proprietor: Cook Biotech Incorporated, West Lafayette, IN 47906 (US)
(72) Inventor: Novak, Tyler, Lafayette, IN 47909 (US); Obermiller, F. Joseph, West Lafayette, IN 47906 (US)
(74) Representative: Stafford, Jonathan Alan Lewis

(56) References cited:
- WO-A1-2015/100238
- WO-A1-2015/187858

## Description

### BACKGROUND

The present disclosure relates to medical technology and in certain embodiments to medical implants that provide electrical stimulation effective to modulate patient tissue growth.

Medical technologies for tissue regeneration often involve implant structures that restore, maintain or improve tissue functions. The implant structures commonly include porous scaffolds made from synthetic or naturally-occurring polymers and into which patient tissue can grow. Challenges faced in the field of tissue engineering include generating implants that lead to the development of desired native patient tissue structures over a period of time after implantation. Variables such as scaffold material, pore size, and the inclusion of chemical signals such as growth factors and other bioactive molecules have been widely explored in attempts to provide implants with the requisite functions.

Despite developments to date in fields related to tissue regeneration, there remain needs for implants, and methods for their preparation and use, that beneficially develop patient tissue when implanted. In certain aspects, embodiments of the present disclosure are addressed to these needs.

WO2015/100238 A1 describes artificial graft devices and related systems and methods for providing cardiovascular bypass for mammalian subjects.

WO2015/187858 A1 describes methods and devices for surgical pretreatment, for example using bioelectric devices that comprise a multi-array matrix of biocompatible microcells and a fluid such as a conductive fluid or cream.

### SUMMARY

In certain aspects, the present disclosure relates to products that include a graft material and a source of galvanically generated electric current associated with the graft material. The source of electric current can include a galvanic couple structure that includes first and second metals that differ from one another, with the galvanic couple structure attached to the graft material. Accordingly, some embodiments herein provide a self-powering electrostimulative graft product for treating a patient. The graft product includes a porous graft matrix material receptive to ingrowth of new tissue when implanted in the patient. A galvanic couple structure is attached to the porous matrix material and includes a cathode comprised of a first metal, preferably a biodegradable metal, and an anode comprised of a second metal, preferably a biodegradable metal, with the first metal being different from the second metal. The galvanic couple structure can be operable to generate electric current in a path between the anode and the cathode, with the path extending through amounts of the porous matrix material positioned between the anode and the cathode.

In other embodiments, provided are self-powering electrostimulative graft products for treating a patient. The graft products include a porous graft matrix material receptive to ingrowth of new tissue when implanted in the patient, the porous graft matrix material at least in part in the form of a tube. A galvanic couple structure is attached to the porous matrix material and includes a cathode comprised of a first metal and an anode comprised of a second metal, the first metal being different from the second metal. The galvanic couple structure can be operable to generate electric current in a path between the anode and the cathode, with the path extending in an axial direction along the tube. The first and second metals are preferably biodegradable metals.

In still further embodiments, provided are implantable graft products that include a laminate structure including a plurality of sheets of porous graft matrix material laminated to one another. A first electrode comprised of a first metal is captured within the laminate structure between adjacent sheets of said plurality of sheets. A second electrode comprised of a second metal is captured within the laminate structure between adjacent sheets of said plurality of sheets, with the second electrode spaced from the first electrode. The first metal is different from said second metal, and the first biodegradable metal and the second biodegradable metal form a galvanic couple. The first and second metals are preferably biodegradable metals.

Further disclosed, but not forming part of the invention are methods for generating new tissue growth in a patient. The methods include generating an electric current through a porous graft matrix material by a self-powering galvanic couple structure and during a period in which new patient tissue grows into the porous graft matrix material. The anode(s) and cathode(s) of the galvanic couple structure can be attached to the porous graft matrix material in some embodiments. In other embodiments, an anode(s) and cathode(s) can be attached to one or more separate grafts implanted in conjunction with the porous graft matrix material.

Additional embodiments herein relate to methods for preparing self-powering electrostimulative graft products, and methods for using electrostimulative graft products.

In embodiments set forth in this Summary above and elsewhere herein, the porous graft matrix material can include collagen; and/or the porous graft matrix material can include one or more decellularized membranous tissue sheets; and/or the porous graft matrix material can include decellularized tissue selected from submucosal tissue, dermal tissue, pericardial tissue, amnion tissue, peritoneal tissue, or fascia tissue; and/or the first biodegradable metal and the second biodegradable metal exhibit a standard electrode potential difference of at least about 0.05V, and preferably in the range of about 0.05 volts (V) to about 3 V.

Additional embodiments of the present disclosure, as well as features and advantages thereof, will be apparent from the descriptions herein.

### BRIEF DESCRIPTION OF THE DRAWINGS

FIG. 1 provides a plan view of an electrostimulative graft.
FIG. 2 provides a cross-sectional view of one embodiment of the graft of FIG. 1 taken along line 2-2 and viewed in the direction of the arrows.
FIG. 3 provides a cross-sectional view of one embodiment of the graft of FIG. 1 taken along line 3-3 and viewed in the direction of the arrows.
FIG. 4 provides a cross-sectional view of another embodiment of the graft of FIG. 1 taken along line 2-2 and viewed in the direction of the arrows.
FIG. 5 provides a cross-sectional view of another embodiment of the graft of FIG. 1 taken along line 2-2 and viewed in the direction of the arrows.
FIG. 6 provides a cross-sectional view of another embodiment of the graft of FIG. 1 taken along line 2-2 and viewed in the direction of the arrows.
FIG. 7 provides a cross-sectional view of another embodiment of a graft of FIG. 1 taken along line 2-2 and viewed in the direction of the arrows.
FIG. 8 provides a cross-sectional view of another embodiment of a graft of FIG. 1 taken along line 2-2 and viewed in the direction of the arrows.
FIG. 9 provides a perspective view of a tubular electrostimulative graft.
FIG. 10 provides a longitudinal cross-sectional view of the graft of FIG. 9.
FIG. 11 provides a perspective view of another tubular electrostimulative graft.
FIG. 12 provides a longitudinal cross-sectional view of the graft of FIG. 11.
FIG. 13 provides a cross-sectional view of one embodiment of an electrode encapsulated by an electrically insulating biodegradable coating.

### DETAILED DESCRIPTION

While the present invention may be embodied in many different forms, for the purpose of promoting an understanding of the principles of the present invention, reference will now be made to embodiments, some of which are illustrated in the drawings, and specific language will be used to describe the same. It will nevertheless be understood that no limitation of the scope of the invention is thereby intended. Any alterations and further modifications in the described embodiments and any further applications of the principles of the present invention as described herein are contemplated as would normally occur to one skilled in the art to which the invention relates. Additionally, in the detailed description below, numerous alternatives are given for various features related to the composition or size of materials, or to modes of carrying out methods. It will be understood that each such disclosed alternative, or combinations of such disclosed alternatives, can be combined with the more generalized features discussed in the Summary above, or set forth in the Listing of Certain Embodiments below, to provide additional disclosed embodiments herein.

As disclosed above, aspects of the present disclosure relate to self-powering electrostimulative grafts that include a graft material and a galvanic couple structure associated with the graft material, as well as methods for their preparation and use. In these regards, as used herein, the term "galvanic couple structure" refers to two different materials that are spaced from one another and that, when electrically connected by an electrically conductive medium such as an ion-containing medium, generate electric current between the different materials from spontaneous redox reactions.

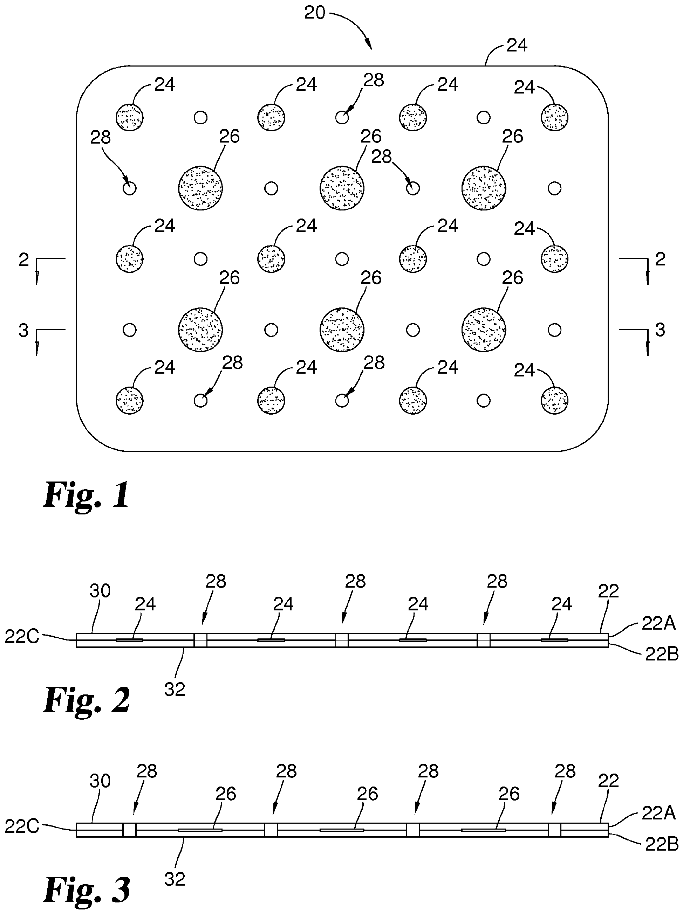

With reference now to the drawings, Figure 1 provides a plan view for an electrostimulative graft 20 which can be used in numerous embodiments of the present disclosure. Graft 20 includes a graft body 22, preferably comprised of a porous graft matrix material receptive to ingrowth of new tissue when implanted in the patient. A variety of naturally-derived or synthetic materials, or their combinations, may be used in the graft body 22, including those discussed hereinbelow. Graft 20 also includes at least one cathode 24, and preferably a plurality of cathodes 24 as illustrated, and at least one anode 26, and preferably a plurality of anodes 26 as illustrated. Cathode(s) 24 and anode(s) 26 form one or more galvanic couples associated with the graft body 22, with cathode(s) 24 and anode(s) preferably attached to the graft body 22. As illustrated, cathode(s) 24 and anode(s) 26 are spaced from one another on the graft body 20, and separated by material of the graft body 20. In discussions below, the cathode(s) and anode(s) will sometimes be referred to together as "the electrodes". In some forms, graft 20 also includes a plurality of thru-holes 28, which can allow the passage of fluid through the graft body 22. As will be discussed below, the lateral arrangement of the cathode(s) and anode(s) of the graft 20 with respect to one another shown in Fig. 1, as well as other lateral arrangements, can be used while locating the electrodes on the surface of the graft body 22, within the graft body 22, or combinations thereof.

With reference now to Figs. 2 and 3, along with Fig. 1, shown is one illustrative embodiment of a graft 20, where the electrodes are located within the graft body 22. Fig. 2 provides a cross-sectional view of one embodiment of the graft layout of Fig. 1, taken along line 2-2 and viewed in the direction of the arrows. Fig. 3 provides a cross-sectional view of this embodiment taken along line 3-3 of Fig. 1 and viewed in the direction of the arrows. Shown are the cathodes 24 and anodes 26 embedded within the graft body 22, with amounts of the material of the graft body 22 surrounding the cathodes 24 and anodes 26 on all sides. In this illustrated embodiment, the graft body 22 is a laminate, with a first layer or layers 22A of graft material occurring toward a first surface 30 of graft body 22 and a second layer or layers 22B of graft material occurring toward a second surface 32 of the graft body opposite the first surface 30. A laminate interface 22C occurs between layer(s) 22A and layer(s) 22B, with cathodes 24 and anodes 26 captured between layer(s) 22A and layer(s) 22B at the interface. Interface 22C is preferably a bonded interface. Suitable bonding techniques are discussed hereinbelow.

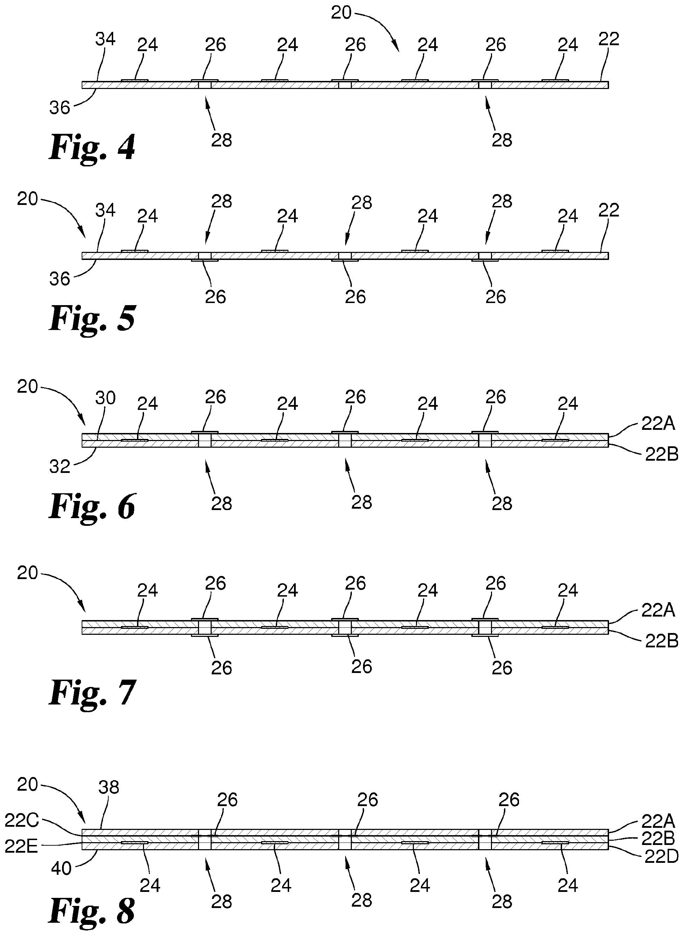

Referring now to Fig. 4, shown is another illustrative embodiment of a graft 20, where the electrodes are located on an outer surface 34 of the graft body 22. Fig. 4 provides a cross-sectional view of this embodiment of the graft layout of Fig. 1, taken along line 2-2 and viewed in the direction of the arrows. Cathodes 24 and anodes 26 are bonded or otherwise attached to the same outer surface 34 of graft body 22 in this embodiment. Segments of outer surface 34 separate the cathodes 24 and anodes 26. Impregnation of graft body 22 with a conductive fluid, for example body fluid, provides electrical communication between cathodes 24 and anodes 26, causing electric current flow between cathodes 24 and anodes 26 that can serve to electrostimulate tissue development within graft body 22 and adjacent graft body 22. In this embodiment, surface 36 opposite surface 34 is free of any cathodes 24 or anodes 26.

Shown in Fig. 5 is another illustrative embodiment of a graft 20, where the electrodes are located on surfaces of the graft body 22, with the cathodes 24 located on a first outer surface 34 and the anodes 26 located on a second outer surface 36 opposite the first surface. Fig. 5 provides a cross-sectional view of this embodiment of the graft layout of Fig. 1, taken along line 2-2 and viewed in the direction of the arrows. In this embodiment, a thickness of the graft body 22 separates the cathodes 24 and anodes 26. In this manner, the thickness of the graft body 22 provides a barrier against any risk of direct contact of cathodes 24 and anodes 26 with each other when graft 20 is implanted. Such direct contact would interrupt a desired communication of current through conductive fluid, for example body fluid, within and surrounding graft body 22 upon implantation. Also in the embodiment of Fig. 5, the cathodes 24 and anodes 26 are not aligned (or not in registry) with one another through the thickness of the graft body 22, establishing a shortest distance between cathodes 24 and anodes 26 along a line that travels diagonally through the thickness of graft body 22. It is contemplated in other, related embodiments, that the cathodes 24 and anodes 26 can be partially or completely aligned with one another through the thickness of the graft body 22. In still other embodiments, combinations of partially, completely and/or non-aligned electrodes through the thickness of the graft body 22 can be used. Also, in any of these embodiments, the positions of the cathodes 24 and anodes 26 can be interchanged with one another, or combinations of cathodes 24 on both sides 30 and 32 and/or of anodes 26 on both sides 30 and 32 can be included.

Fig. 6 shows another illustrative embodiment of a graft 20, in which some electrodes are located within the graft body 22 and some electrodes are located on an outer surface at one side 30 of the graft body 22. Fig. 6 provides a cross-sectional view of this embodiment of the graft layout of Fig. 1, taken along line 2-2 and viewed in the direction of the arrows. In this embodiment, a partial thickness of the graft body 22 separates the cathodes 24 and anodes 26. As in the embodiment of Fig. 5, this thickness of the graft body 22 provides a barrier against any risk of direct contact of cathodes 24 and anodes 26 with each other when graft 20 is implanted. Again, such direct contact would interrupt a desired communication of current through conductive fluid, for example body fluid, within and surrounding graft body 22 upon implantation. Also in the embodiment of Fig. 6, the cathodes 24 and anodes 26 are not aligned (or not in registry) with one another through the thickness of the graft body 22, establishing a shortest distance between cathodes 24 and anodes 26 along a line that travels diagonally through the partial thickness of graft body 22. It is contemplated in other, related embodiments, that the cathodes 24 and anodes 26 can be partially or completely aligned with one another through the thickness of the graft body 22. In still other embodiments, combinations of partially, completely and/or non-aligned electrodes through the partial thickness of the graft body 22 can be used. Also, in any of these embodiments, the positions of the cathodes 24 and anodes 26 can be interchanged with one another, or combinations of outer and inner cathodes 24 and/or of outer and inner anodes 26 can be included.

Referring now to Fig. 7, shown is an illustrative embodiment of a graft 20, in which some electrodes are located within graft body 22, some electrodes are located on a first outer surface at one side 30 of the graft body 22, and some electrodes are located on a second outer surface at another side 32 of the graft body 22. Fig. 7 provides a cross-sectional view of this embodiment of the graft layout of Fig. 1, taken along line 2-2 and viewed in the direction of the arrows. In this embodiment, as in the embodiment of Fig. 6, a partial thickness of the graft body 22 separates the cathodes 24, which are located within graft body 22, and anodes 26, which are located on opposite outer surfaces 30 and 32 of graft body 22. This thickness of the graft body 22 provides a barrier against any risk of direct contact of cathodes 24 and anodes 26 with each other when graft 20 is implanted. As before, such direct contact would interrupt a desired communication of current through conductive fluid, for example body fluid, within and surrounding graft body 22 upon implantation. Also in the embodiment of Fig. 7, the cathodes 24 and anodes 26 are not aligned (or not in registry) with one another through the thickness of the graft body 22, establishing a shortest distance between cathodes 24 and anodes 26 along a line that travels diagonally through the partial thickness of graft body 22. However, the anodes 26 occurring on opposite sides 30 and 32 of the graft body 22 are aligned (or in registry with) one another through the thickness of the graft body 22. It is contemplated in other, related embodiments, that the cathodes 24 and anodes 26 can be partially or completely aligned with one another through the thickness of the graft body 22. In still other embodiments, combinations of partially, completely and/or non-aligned electrodes through the partial thickness of the graft body 22 can be used. Also, in any of these embodiments, the positions of the cathodes 24 and anodes 26 can be interchanged with one another, or combinations of outer and inner cathodes 24 and/or of outer and inner anodes 26 can be included.

Shown in Fig. 8 is still another illustrative embodiment of a graft 20, in which electrodes are located within the graft body 22 at different levels through the thickness of the graft body between a first outer surface 38 and a second outer surface 40 opposite the first outer surface 38. Fig. 8 provides a cross-sectional view of this embodiment of the graft layout of Fig. 1, taken along line 2-2 and viewed in the direction of the arrows. In this embodiment, as some prior-discussed embodiments, a partial thickness of the graft body 22 separates the cathodes 24, which are located within graft body 22, and anodes 26, which are also located within the graft body. This thickness of the graft body 22 provides a barrier against any risk of direct contact of cathodes 24 and anodes 26 with each other when graft 20 is implanted. As before, such direct contact would interrupt a desired communication of current through conductive fluid, for example body fluid, within and surrounding graft body 22 upon implantation. Also in the embodiment of Fig. 8, the cathodes 24 and anodes 26 are not aligned (or not in registry) with one another through the thickness of the graft body 22, establishing a shortest distance between cathodes 24 and anodes 26 along a line that travels diagonally through the partial thickness of graft body 22. It is contemplated in other, related embodiments, that the cathodes 24 and anodes 26 can be partially or completely aligned with one another through the thickness of the graft body 22. In still other embodiments, combinations of partially, completely and/or non-aligned electrodes through the partial thickness of the graft body 22 can be used. Also, in any of these embodiments, the positions of the cathodes 24 and anodes 26 can be interchanged with one another, or combinations cathodes 24 and/or anodes 26 at different levels within the thickness of the graft body 22 can be included. The graft 20 illustrated in Fig. 8 is a laminate including layer(s) 22A and layer(s) 22B as in some of the embodiments discussed above, as well as layer(s) 22D. This provides a further laminate interface 22D at which some of the electrodes (e.g. cathodes 24) can be positioned.

Electrostimulative grafts of the present disclosure can have any suitable three-dimensional form. For example, grafts discussed in conjunction with Figs. 1-8 above can have a non-tubular form, such as a sheet form. In other embodiments, electrostimulative grafts of the present disclosure have at least a portion that forms a tube, and potentially are tubular grafts. For example, partially or completely tubular grafts can have electrode positioning on surface(s) and/or within thicknesses of the grafts that are the same as those discussed in conjunction with Figs. 1-8, in certain embodiments. Such grafts may or may not have thru-holes 28 as depicted in Figs. 1-8. As well, in some forms, such grafts may have electrode positioning so that the galvanic couple provides a current that extends axially (along the long axis of) the tube or tube portion of the graft.

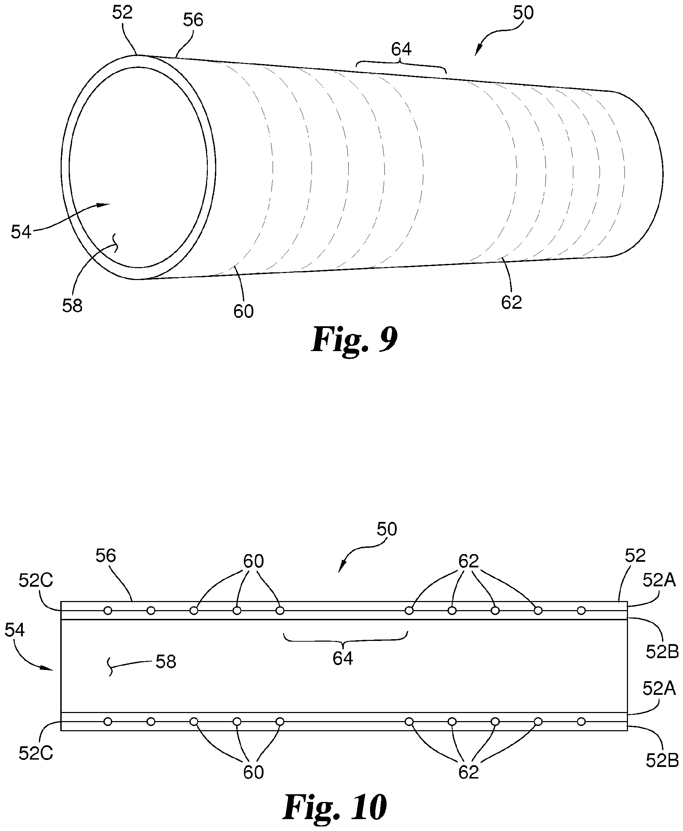

With reference now to Figs. 9 and 10, shown is one illustrative electrostimulative graft 50 in the form of a tube. Fig. 9 provides a perspective view of graft 50, and Fig. 10 provides a cross-sectional view taken along the longitudinal axis of graft 50 of Fig. 9. Graft 50 includes a tubular graft wall 52 defining an inner lumen 54. In this illustrated embodiment, the graft wall 52 is a laminate, with a first layer or layers 52A of graft material occurring toward an outer surface 56 of graft wall 52 and a second layer or layers 52B of graft material occurring toward a luminal surface 58 of the graft wall opposite the outer surface 56. A laminate interface 52C occurs between layer(s) 52A and layer(s) 52B. A cathode 60, preferably in the form of a helical wire as shown, and an anode 62, also preferably in the form of a helical wire as shown, are captured between layer(s) 52A and layer(s) 52B at the interface 52C. Interface 52C is preferably a bonded interface. Suitable bonding techniques are discussed hereinbelow. In this illustrated embodiment, the cathode 60 occurs to a first longitudinal side of the graft 50 and the anode 62 occurs to a second longitudinal side of the graft 50, with an intermediate segment 64 of the graft wall 52 separating the central-most end of the cathode 60 from the central-most end of the anode 62 along the length of the tubular graft wall 52. In this form, the galvanic couple will generate current that travels axially along the tubular graft. As is discussed further below, tubular grafts such as those depicted in Figs. 9 and 10 can in some modes be used as nerve cuffs or wraps to stimulate the growth of nerve tissue.

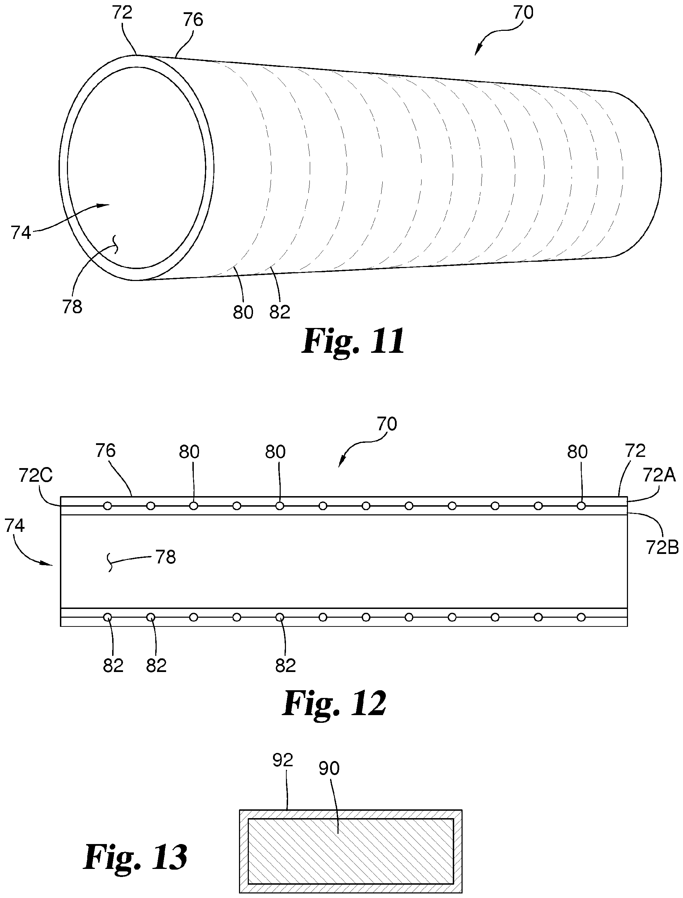

Referring now to Figs. 11 and 12, shown is another illustrative electrostimulative graft 70 in the form of a tube. Fig. 11 provides a perspective view of graft 70, and Fig. 12 provides a cross-sectional view taken along the longitudinal axis of graft 70 of Fig. 19. Graft 70 includes a tubular graft wall 72 defining an inner lumen 74. In this illustrated embodiment, the graft wall 72 is a laminate, with a first layer or layers 72A of graft material occurring toward an outer surface 76 of graft wall 72 and a second layer or layers 72B of graft material occurring toward a luminal surface 78 of the graft wall opposite the outer surface 76. A laminate interface 72C occurs between layer(s) 72A and layer(s) 72B. A cathode 80, preferably in the form of a helical wire as shown, and an anode 82, also preferably in the form of a helical wire as shown, are captured between layer(s) 72A and layer(s) 72B at the interface 72C. Interface 72C is preferably a bonded interface, with suitable bonding techniques discussed hereinbelow. In this illustrated embodiment, the cathode 80 and the anode 82 are provided as overlapped helical wires positioned so that the turns of the wires of cathode 80 and the turns of the wires of anode 82 occur alternately, but do not directly contact one another, along the length of the graft body 72. In this manner, a plurality of galvanic couple structures are provided along the length of the tubular graft 70. Each of these galvanic couple structures will generate current that extends axially along the tubular graft. As is discussed further below, tubular grafts such as those depicted in Figs. 11 and 12 can in some modes be used as nerve cuffs or wraps to stimulate the growth of nerve tissue.

While the grafts shown in Figs. 9-12 are shown as completely circumferential tubes, it is also contemplated that similar grafts can be prepared having a generally tubular shape but including a longitudinal slit extending the length of the grafts. Such split grafts may be more readily implanted over longitudinally-extending structures such as vessels or nerves, since a side-mount approach may be used in which the patient tissue structure is passed through the longitudinal slit into the lumen region of the graft.

### Graft Materials

In some forms, the graft material of the electrostimulative grafts herein will include one or more intact segments of decellularized collagenous tissue membrane. Suitable materials for incorporation in any of the embodiments herein can be provided by membranous collagenous extracellular matrix (ECM) materials. For example, suitable membranous ECM materials include as examples those comprising submucosa, renal capsule membrane, dermal collagen, dura mater, pericardium, fascia lata, serosa, subserous fascia, amnion, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. These or other ECM materials can be characterized as membranous tissue layers or sheets harvested from a source tissue and decellularized. These membranous tissue sheets can have a porous matrix comprised of a network of collagen fibers, wherein the network of collagen fibers preferably retains an inherent network structure from the source tissue. In particular aspects, collagenous matrices comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source, and decellularizing the matrix before or after such delaminating . For additional information as to some of the materials useful in the present invention, and their isolation and treatment, reference can be made, for example, to U.S. Patent Nos. 4,902,508, 5,554,389, 5,993,844, 6,206,931, 6,099,567, 8,541,372 and 9,044,455.

Submucosa-containing or other ECM tissue, when used in the invention, is preferably highly purified, for example, as described in U.S. Patent No. 6,206,931 to Cook et al. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of any ECM tissue used in the present invention.

Submucosa-containing or other membranous ECM tissue material may retain one or more growth factors native to the source tissue for the tissue material, such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in embodiments herein may retain other bioactive agents native to the source tissue, such as but not limited to proteins, glycoproteins, proteoglycans, and glycosaminoglycans. For example, ECM materials may include native heparin, native heparin sulfate, native hyaluronic acid, native fibronectin, native cytokines, and the like. Thus, generally speaking, a submucosa or other ECM material may retain one or more native bioactive components from the source tissue that induce, directly or indirectly, a cellular response such as a change in cell morphology, proliferation, growth, protein or gene expression. In some forms, in addition to providing bioactive signals to the graft material, these or other non-collagen substances retained from the source tissue can affect the conductivity of the graft material, especially when wetted (e.g. after implant by body fluid) and especially when the retained substances are ionically charged, and thereby affect the electrostimulative properties of the graft.

Submucosa-containing or other ECM materials can be derived from any suitable organ or other tissue source, usually sources containing connective tissues. The ECM materials processed for use in the invention will typically be membranous tissue sheets that include abundant collagen, most commonly being constituted at least about 80% by weight collagen on a dry weight basis. Such naturally-derived ECM materials will for the most part include collagen fibers that are non-randomly oriented, for instance occurring as generally uniaxial or multi-axial but regularly oriented fibers. When processed to retain native bioactive factors, the ECM material can retain these factors interspersed as solids between, upon and/or within the collagen fibers. Particularly desirable naturally-derived ECM materials for use in the invention will include significant amounts of such interspersed, non-collagenous solids that are readily ascertainable under light microscopic examination with appropriate staining. Such non-collagenous solids can constitute a significant percentage of the dry weight of the ECM material in certain inventive embodiments, for example at least about 1%, at least about 3%, and at least about 5 % by weight in various embodiments of the invention.

A submucosa-containing or other ECM material used in embodiments herein may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the formation of new blood vessels into the materials. Methods for measuring in vivo angiogenesis in response to biomaterial implantation have recently been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

Further, in addition or as an alternative to the inclusion of such native bioactive components retained from a source tissue, non-native bioactive components such as those synthetically produced by recombinant technology or other methods (e.g., genetic material such as DNA), may be incorporated into the ECM material. These non-native bioactive components may be naturally-derived or recombinantly produced proteins that correspond to those natively occurring in an ECM tissue, but perhaps of a different species. These non-native bioactive components may also be drug substances. Illustrative drug substances that may be added to materials include, for example, anti-clotting agents, e.g. heparin, antibiotics, anti-inflammatory agents, thrombus-promoting substances such as blood clotting factors, e.g., thrombin, fibrinogen, and the like, and anti-proliferative agents, e.g. taxol derivatives such as paclitaxel. The non-native bioactive component(s) can also be cells, including for example stem cells, which can be attached to and/or cultured on the ECM of electrostimulative grafts prior to implantation if desired. These and/or other non-native bioactive components can be incorporated into and/or onto ECM material in any suitable manner, for example, by surface treatment (e.g., spraying) and/or impregnation (e.g., soaking), just to name a few. Also, these substances may be applied to the ECM material in a premanufacturing step, immediately prior to the procedure (e.g., by soaking the electrostimulative graft in a solution containing a suitable antibiotic such as cefazolin), or during or after engraftment of the electrostimulative graft in the patient.

In certain embodiments, the electrostimulative graft can include a laminate of two or more individual layers of membranous ECM material (e.g., 2 or more layers bonded together). The total thickness of such a construct can in some forms be more than about 400 microns, or more than about 600 microns, or more than about 800 microns, or more than about 1,000 microns, or more than about 1,200 microns, or more than about 1,500 microns but typically less than about 2,000 microns. In certain aspects, the thickness of such a construct is in the range of about 200 microns to about 4,000 microns. Also in certain aspects, 2 to about 20 layers of membranous ECM tissue material are bonded in a laminate construct of an electrostimulative graft.

Suitable bonding techniques in forming laminate constructs include chemical crosslinking and techniques other than chemical crosslinking, or combinations thereof. Techniques other than chemical cross-linking include vacuum pressing, lyophilization, or other dehydrothermal bonding conditions and/or the use of an adhesive, glue or other bonding agents. Suitable bonding agents may include, for example, collagen gels or pastes, gelatin, fibrin glues, or other agents including reactive monomers or polymers, for example cyanoacrylate adhesives. Bonding by chemical crosslinking can achieved using chemical cross-linking agents, such as glutaraldehyde, formaldehyde, epoxides, genipin or derivatives thereof, carbodiimide compounds, polyepoxide compounds, or other similar agents. The combination of dehydration-induced bonding and chemical crosslinking is used in certain embodiments. In embodiments, as discussed above, where an electrode or electrodes are to be located within a thickness of a graft, such electrode(s) can be positioned between layers to form the laminate prior to bonding the layers to one another.

A variety of dehydration-induced bonding methods can be used to fuse and thereby laminate layers of membranous ECM materials together. In one preferred embodiment, multiple layers of the membranous ECM material are compressed under dehydrating conditions. The term "dehydrating conditions" can include any mechanical or environmental condition which promotes or induces the removal of water from the multi-layered medical material. To promote dehydration of the compressed material, at least one of the two surfaces compressing the matrix structure can be water permeable. Dehydration of the material can optionally be further enhanced by applying blotting material, heating the matrix structure or blowing air, or other inert gas, across the exterior of the compressing surfaces. Particularly useful methods of dehydration bonding the ECM layers to one another include lyophilization, e.g. freeze-drying or evaporative cooling conditions, vacuum pressing, oven drying and/or air drying.

ECM materials for use in making electrostimulative grafts can be processed using methods that decrease the content of undesired components of the source tissue such as cells, nucleic acid, lipids and/or immunoglobulins such as IgA, while retaining substantial levels of desired components from the source tissue such as growth factor(s) (e.g. Fibroblast Growth Factor-2), proteoglycans and/or glycosaminoglycans (GAGs). Such treatments can be performed with detergent, basic medium, liquid organic solvent, and/or disinfecting solution, for example as described in U.S. Patent No. 8,192,763 issued June 5, 2012, the disclosure of which is specifically incorporated herein by reference in its entirety.

While in certain embodiments the porous graft material can include or be an extracellular matrix material as discussed above, in other embodiments the porous graft material can be or include a synthetic graft material, for example a synthetic polymeric graft material. Such synthetic polymeric or other graft materials can be biodegradable or non-biodegradable, and are preferably biodegradable. Suitable biodegradable polymers for these purposes include, for example, aliphatic polyesters, poly (amino acids), copoly (ether-esters), polyalkylenes oxalates, polyamides, tyrosine derived polycarbonates, poly (iminocarbonates), polyorthoesters, polyoxaesters, polyamidoesters, polyoxaesters containing amine groups, poly (anhydrides), polyphosphazenes, and combinations thereof. Biodegradable aliphatic polyesters include, but are not limited to, homopolymers and copolymers of lactide (which includes lactic acid, D-, L- and meso lactide), glycolide (including glycolic acid), epsilon-caprolactone, p-dioxanone (1,4-dioxan-2-one), trimethylene carbonate (1,3-dioxan-2-one), alkyl derivatives of trimethylene carbonate, and polymer blends thereof. These or other synthetic polymers can be molded, cast or otherwise processed to form a porous graft matrix for use in electrostimulative grafts herein. The porous graft matrix can be a laminate of layers as discussed hereinabove, or in other embodiments can be a unitary, continuous matrix. In embodiments, as discussed above, where an electrode or electrodes are to be included within a thickness of the graft material, any suitable technique can be used to locate and embed the electrode(s) within the thickness. These include, for example, casting or molding the polymeric matrix forming material around the electrode(s) to be embedded within the porous graft material thickness.

In preferred forms the porous graft matrix material is biodegradable. In addition or alternatively, the porous graft matrix material, whether being a naturally-derived material such as an ECM discussed above, or a synthetic polymeric material as discussed above, will be bioremodelable such that the graft matrix material is degraded as new tissue of the patient grows into the graft matrix material. Still further, in some forms, the electrostimulative grafts can be completely biodegradable after implantation in a patient, with both the electrodes and the porous graft matrix material ultimately being completely degraded and eliminated from the site of implantation.

### Electrode Structure and Formation

While Figs. 1-8 depict disc-shaped electrodes and Figs. 9-12 depict wire electrodes, it is contemplated that any suitable electrode shape can be employed. These include for example strips, wires, foils, sheets, meshes, or other shapes. As well, electrodes can be formed in any suitable fashion. As examples, they can be cut or stamped from sheets, printed (e.g. as with a conductive ink) or plated onto the graft material or another substrate material to be included in the graft. Further, individual electrodes can be monolithic structures, or can be stacks or laminates (e.g. of metal foil layers) or masses of directly contacting particles of the cathode or anode material (e.g. deposited particles that are captured at the interface of a laminate graft structure as discussed above). These and other arrangements are contemplated as being within the scope of the embodiments disclosed herein.

The electrodes of the electrostimulative grafts can be generally rigid structures that hold their shape on implantation, can be resilient structures that flex upon being subjected to external force (e.g. forces experienced by patient movements after implant) and return to their original shape upon removal of the external force, or can be conformable by plastic deformation against patient soft tissue or patient hard tissue (e.g. bone) upon implantation. Materials selection and electrode geometries can be controlled to impart these or other desired physical properties to the electrodes. Combinations of electrodes with different physical properties, such as those discussed above, can also be used.

Still further, in some embodiments, some or all of the electrodes of the graft can be temporarily electrically insulated by an insulative biodegradable material, for example one that is impervious to body fluids. For example, such an electrically insulative biodegradable material can be used to coat and encapsulate the electrode(s). Fig. 13 provides a cross-sectional view of one such illustrative embodiment, in which the electrode 90 (which can for example serve as some or all of the cathode(s) 24, 60 or 80 and/or some or all of the anodes 26, 62 or 82 discussed hereinabove) has an encapsulating coating 92 of an insulative biodegradable material. After a duration of time following implantation, the insulative biodegradable material degrades and allows body fluid contact with the electrode(s), providing electrical communication between anode(s) and cathode(s) of a galvanic structure. In this manner, a delayed initiation of electrostimulation by one, more than one, or all of the galvanic couples provided by the electrostimulative graft can be provided. In some forms, a plurality of galvanic couple structures can be provided on the graft, with some temporarily electrically insulated by biodegradable material, and some not. The initially non-isolated galvanic couple structures can provide electrostimulation over a first period of time after implantation, and the temporarily electrically isolated galvanic couple structures can provide electrostimulation over a second period of time occurring after the initiation of the first period of time (e.g. partially overlapping the first period of time or occurring after the first period of time). In this manner, staged electrostimulation by differing galvanic couple structures can increase the overall period of electrostimulation provided by the graft after implantation and/or can provide electrostimulation that varies over time in a predetermined pattern.

### Electrode Materials

Any suitable metals can be included in the galvanic couple or couples included in the electrostimulative grafts. The metals can be pure elemental metals or alloys. The metals are desirably biodegradable and desirably form biodegradable materials when subjected to the redox reactions that occur during operation of the galvanic couple to generate current. In preferred forms, the metal of the cathode(s) and the metal of the anode(s) will have a standard electrode potential difference of at least about 0.05V, more preferably at least about 0.1V, and typically in the range of about 0.05V to about 3 V, more typically in the range of about 0.1 V to about 2.4 V. In this regard, as is conventionally known, standard electrode potential is potential of an electrode composed of a substance in its standard state, in equilibrium with ions in their standard states compared to a hydrogen electrode. Desirable metal materials to be included in the cathode(s) and/or anode(s) include, for example, biodegradable metals that are or include iron, magnesium, and/or zinc. In some forms, the electrostimulative grafts will include biodegradable metals for the cathode(s) and anode(s) as set forth in Table 1 below:

**Table 1**

| Cathode | Anode | Standard Potential Difference |
|---|---|---|
| Iron or iron alloy | Magnesium or magnesium alloy | About 0.80-2.40 V |
| Iron or iron alloy | Zinc or zinc alloy | About 0.40-0.80 V |
| Zinc or zinc alloy | Magnesium or magnesium alloy | About 0.60-1.70 V |

The characteristic intensity, spacing and general pattern of the electrical current generated in the electrostimulative graft will be dependent on several factors, including for example the shape of the graft, the size of the graft, the selected anode and cathode materials, the spacing of anode and cathode materials from one another, and the electrical properties of the graft material separating the anode and cathode materials from one another. These parameters can be controlled in the design of an electrostimulative graft to treat a given condition in a patient.

In certain forms, the cathode(s) and anode(s) of the electrostimulative grafts will be spaced from one another on the graft material by a distance of at least about 1 mm, or at least about 2mm, and typically in the range of about 1mm to about 20mm, and more typically in the range of about 2mm to about 15mm. Where multiple anodes and cathodes are included on the electrostimulative graft, they may be included in a regular or irregular pattern. As well, it will be understood that a given cathode may be spaced equidistantly from multiple anodes and thus may participate in a number of generally equivalent galvanic couples, and *vice versa* (see e.g. the electrode patterns of the embodiments of Figs. 1-8). These and other arrangements of the galvanic couple(s) of the electrostimulative grafts will be within the purview of skilled persons in the field given the disclosures herein.

In certain forms, the electrostimulative graft will include at least one, and potentially multiple, galvanic couple structures that, when implanted in the patient or when saturated with physiologic saline (a 0.9% weight/volume solution of sodium chloride in water), will generate a current between the anode and cathode of at least about 0.05 µA, and typically in the range of about 5 µA to about 500 mA, and/or a current density between the anode and cathode of at least about 1 µA/mm², and typically in the range of about 5 µA/mm² to about 500 µA/mm². In addition or alternatively, the electrostimulative graft will include at least one, and potentially multiple, galvanic couple structures that, when implanted in the patient, will generate a voltage potential between the anode and cathode of at least about 0.05 V, and typically in the range of about 0.1 V to 2.40 V, and/or a voltage field strength between the anode and cathode of at least about 10 mV/mm, and typically in the range of about 50 mV/mm to about 1000 mV/mm. It will be understood that the current, current density, voltage potential, or voltage field strength may vary over time after implantation according to one or more factors, including for example tissue formation in and around the implanted graft, biodegradation of implanted graft material occurring between the anode(s) and cathode(s), and galvanic degradation and/or biodegradation of the electrode materials. In desirable forms, the electrostimulative graft will be capable of generating the above-mentioned currents and/or the above-mentioned current densities and/or the above-mentioned voltage potentials and/or the above mentioned voltage field strengths over a period of at least about 6 hours, or at least about 1 day, or at least about 7 days, and in some forms in the range of about 1 day to about 180 days.

The graft material of the electrostimulative graft, in typical embodiments, is generally electrically non-conductive unless wetted with a conductive liquid medium, for example an ion-containing liquid medium such as saline or body fluid. The electrostimulative graft is desirably packaged in a dry condition (e.g. containing less than 10% moisture) within a medical package such as a pouch, foil or tray to prevent appreciable electrical communication between the cathode(s) and anode(s) of the electrostimulative graft during storage. The medical package will in some embodiments be moisture-resistant for these purposes. In certain modes of use, after removal from the medical package, the electrostimulative graft can be implanted in a patient whereupon the graft material becomes wetted with body fluid to establish electrical communication between the cathode(s) and anode(s) and thereby activate the galvanic couple(s) of the graft, or can be wetted with an ion-containing fluid such a physiologic saline or blood or a blood fraction (e.g. autologous to the patient) by a physician or other care provider to activate the galvanic couple(s) prior to implantation in the patient. In many forms, including but not limited to embodiments wherein the graft material is or comprises an ECM material as discussed above, the wetting of the graft material prior to or upon implantation in the patient will render the graft material more supple and conformable to patient tissue.

The electrostimulative grafts disclosed herein can be terminally sterilized (including within medical packages as discussed above) using conventional techniques. For example, the electrostimulative grafts can be terminally sterilized using ethylene oxide and/or using radiation such as E-beam or gas plasma (e.g. Sterrad) processing.

### Uses

Electrostimulative grafts of the present disclosure can be used in the treatment of a wide variety of defects of diseased conditions of soft or hard (e.g. bone) tissues in human or non-human animal (veterinary) patients. The electrostimulation generated by the galvanic couple structures upon implantation will impact the development of patient tissue within and/or around the implanted electrostimulative graft. The electrostimulative graft can be implanted alone in some applications. In other applications, the electrostimulative graft can be implanted along with a separate, secondary implant, for example a secondary porous graft material receptive to tissue ingrowth (e.g. a separate amount of one or more ECM materials as discussed above and/or an amount of one or more synthetic polymeric graft materials as discussed above). The electrostimulative graft can then also electrostimulate regions occupied by the secondary porous graft material or other implant, to facilitate tissue development and/or other tissue regeneration responses within the secondary porous graft material or other implant. In certain forms, patient tissue growth can be stimulated in both the secondary porous graft material and in a porous graft material of the electrostimulative implant.

While the embodiments disclosed in the Figures and certain discussions above include the cathode(s) and anode(s) of the galvanic couple structure attached to the same implantable graft structure, it will be understood that the electrostimulative grafts herein can be provided in multiple (e.g. two, or two or more) discrete graft structures, for example with one (or more) graft structures having attached thereto the cathode(s) of a galvanic couple structure and one (or more) separate graft structures having attached thereto the anode(s) of the galvanic couple structure. The separate graft structures can then be implanted in conjunction with one another to position the cathode(s) and anode(s) in the galvanic couple structure. In some forms, the separate graft structures will have amounts of the porous graft material that can be overlapped with one another between the cathode(s) and anode(s) upon implantation, and the galvanic couple(s) formed on implantation can generate current through and electrostimulate tissue growth into the region between the cathode(s) and anode(s), including into the overlapped amounts of porous graft matrix material. Such separate graft structures in these multi-part grafts can be packaged together (e.g. in packaging as discussed below) to provide a kit with which the physician or other care provider can prepare the electrostimulative graft prior to or upon implantation in the patient.

In certain aspects, the electrostimulative grafts will be used in the repair of nerve tissue in patients. For example, a tubular electrostimulative graft, for example as depicted in Figs. 9-10 or Figs. 11-12, can be used as a nerve cuff in the repair of a severed nerve, such as a severed peripheral nerve. In doing so, the end regions of the severed nerve can be inserted into the lumen of the tubular graft, and the electrostimulation provided by the graft can facilitate axonal growth and functional healing of the severed nerve. It is known that axonal growth occurs from the proximal to the distal stump of severed peripheral nerves. Also, axonal growth occurs toward the cathode during electrostimulation. Thus, in some forms, a cathode-containing end of a tubular or other electrostimulative graft herein (e.g. the tubular graft depicted in Figs. 9-10) can be implanted toward the distal stump, while an anode-containing end of an electrostimulative graft is implanted toward the proximal stump, such that the electrostimulation provided by the graft further facilitates axonal growth toward the distal stump and toward the cathode. As well, a secondary porous graft material receptive to tissue ingrowth, for example a decellularized nerve tissue graft, can be positioned between the proximal and distal stumps, and partially or completely surrounded by the electrostimulative graft. The electrostimulation provided by the electrostimulative graft can facilitate axonal growth through the secondary porous graft material. These and other arrangements to stimulate repair of nerve tissue will be understood.

Additionally, it is known that electrostimulation can facilitate wound healing in soft tissue. Accordingly, in additional embodiments, electrostimulative grafts can be used in the treatment of topical wounds, for example chronic ulcers, or in body wall repair such as hernia repair.

The use of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A self-powering electrostimulative graft product (20) for treating a patient, comprising:
a porous graft matrix material receptive to ingrowth of new tissue when implanted in the patient; and
a galvanic couple structure attached to the porous matrix material and including a cathode (24) comprised of a first metal spaced from an anode (26) comprised of a second metal, the first metal being different from the second metal, and preferably wherein the first metal is a biodegradable metal and the second metal is a biodegradable metal;
the galvanic couple structure operable to generate electric current in a path between the anode and the cathode, the path extending through amounts of the porous matrix material positioned between the anode and the cathode.

2. The graft product (20) of claim 1, wherein:
the porous graft matrix material is at least in part in the form of a tube; and
the path extends in an axial direction along the tube.

3. The graft product (20) of claim 1 or 2, comprising:
a laminate structure including a plurality of sheets (22A,22B) of the porous graft matrix material laminated to one another;
wherein the cathode (24) is captured within the laminate structure between adjacent sheets of said plurality of sheets, preferably wherein the first metal is a biodegradable metal; and
wherein the anode (26) is captured within the laminate structure between adjacent sheets of said plurality of sheets, preferably wherein the second metal is a biodegradable metal.

4. The graft product (20) of any preceding claim, wherein:
a) the porous graft matrix material comprises collagen; and / or
b) wherein the porous graft matrix material comprises one or more decellularized membranous tissue sheets, preferably wherein the decellularized membranous tissue sheets retain one or more native growth factors from a source tissue for the membranous tissue sheets; and / or
c) wherein the porous graft matrix material comprises decellularized tissue selected from submucosal tissue, dermal tissue, pericardial tissue, amnion tissue, peritoneal tissue, or fascia tissue.

5. The graft product (20) of any preceding claim, wherein
a) the first biodegradable metal and the second biodegradable metal exhibit an electrical potential difference of at least about 0.05 V; and, preferably in the range of about 0.05V to about 3 V and / or
b) the first biodegradable metal comprises zinc, magnesium or iron.

6. The graft product (20) of any preceding claim, wherein a) the galvanic couple structure generates an electrical current between the cathode (24) and the anode (26) in the range of about 5 µA to about 500 mA when the graft is saturated in physiologic saline; and / or b) the galvanic couple has an anode (26) and a cathode (24) having a standard electrode potential difference of at least about 0.05 V.

7. The graft product (20) of any preceding claim, wherein the anode (26) and the cathode (24) are sized and configured to degrade within about 180 days after implantation of the tissue graft in a patient.

8. The graft product (20) of any preceding claim, wherein the cathode (24) is: a) spaced from the anode (26) by a distance of at least 1mm; or b) spaced a distance of about 1 mm to about 20 mm from the anode (26).

9. The graft product (20) of any preceding claim, wherein the cathode (24) has a helical shape and/or wherein the anode (26) has a helical shape; optionally wherein the anode and cathode both have a helical shape, and wherein helical turns of the anode are positioned between and extend in a generally parallel fashion to helical turns of the cathode.

10. The graft product (20) of any preceding claim, wherein the porous graft matrix material is biodegradable and/or wherein the porous graft matrix material is bioremodelable.

11. The graft product (20) of any preceding claim, wherein at least one of the anode (26) and the cathode (24) is electrically insulated by a biodegradable insulative polymer, preferably wherein both the anode and the cathode are electrically insulated by a biodegradable insulative polymer.

12. The graft product (20) of any preceding claim, comprising a plurality of said galvanic couple structures.

13. A graft product (20) of any preceding claim, wherein the galvanic couple includes:
(i) a cathode (24) comprising iron or an iron alloy and an anode (26) comprising magnesium or a magnesium alloy; or
(ii) a cathode (24) comprising iron or an iron alloy and an anode (26) comprising zinc or a zinc alloy; or
(iii) a cathode (24) comprising zinc or a zinc alloy and an anode (26) comprising magnesium or a magnesium alloy.

14. A graft product (20) according to any preceding claim, sterilely enclosed within medical packaging, preferably wherein the medical packaging is moisture-resistant packaging.

15. The graft product (20) of claim 14, in dry condition within the medical packaging.

## Patentansprüche

1. Elektrostimulatives Transplantatprodukt mit eigener Leistungsversorgung (20) zum Behandeln eines Patienten, umfassend:
ein poröses Transplantatmatrixmaterial, aufnahmefähig zum Einwachsen von neuem Gewebe, wenn es in den Patienten implantiert wird; und
eine galvanische Kopplungsstruktur, angebracht an das poröse Matrixmaterial und enthaltend eine Kathode (24), bestehend aus einem ersten Metall, beabstandet von einer Anode (26), bestehend aus einem zweiten Metall, wobei das erste Metall von dem zweiten Metall verschieden ist, und wobei vorzugsweise das erste Metall ein bioabbaubares Metall ist und das zweite Metall ein bioabbaubares Metall ist;
wobei die galvanische Kopplungsstruktur funktionsfähig ist, elektrischen Strom in einem Weg zwischen der Anode und der Kathode zu erzeugen, wobei der Weg sich durch Mengen des zwischen der Anode und der Kathode positionierten porösen Matrixmaterials erstreckt.

2. Transplantatprodukt (20) nach Anspruch 1, wobei:
das poröse Transplantatmatrixmaterial mindestens teilweise in der Form eines Schlauchs ist; und
der Weg sich in einer axialen Richtung entlang des Schlauchs erstreckt.

3. Transplantatprodukt (20) nach Anspruch 1 oder 2, umfassend:
eine Laminatstruktur, enthaltend eine Vielzahl von Folien (22A, 22B) des porösen Transplantatmatrixmaterials, die aneinander laminiert sind;
wobei die Kathode (24) innerhalb der Laminatstruktur zwischen aneinander angrenzenden Folien der Vielzahl von Folien aufgenommen ist, wobei vorzugsweise das erste Metall ein bioabbaubares Metall ist; und
wobei die Anode (26) innerhalb der Laminatstruktur zwischen aneinander angrenzenden Folien der Vielzahl von Folien aufgenommen ist, wobei vorzugsweise das zweite Metall ein bioabbaubares Metall ist.

4. Transplantatprodukt (20) nach einem der vorstehenden Ansprüche, wobei:
a) das poröse Transplantatmatrixmaterial Kollagen umfasst; und/oder
b) wobei das poröse Transplantatmatrixmaterial eine oder mehrere Folien von dezellularisiertem membranartigem Gewebe umfasst, wobei vorzugsweise die Folien von dezellularisiertem membranartigem Gewebe einen oder mehrere native Wachstumsfaktoren aus einem Ursprungsgewebe für die Folien von membranartigem Gewebe zurückbehalten; und/oder
c) wobei das poröse Transplantatmatrixmaterial dezellularisiertes Gewebe umfasst, ausgewählt aus submukösem Gewebe, Hautgewebe, Herzbeutelgewebe, Amniongewebe, Bauchfellgewebe oder Fasziengewebe.

5. Transplantatprodukt (20) nach einem der vorstehenden Ansprüche, wobei
a) das erste bioabbaubare Metall und das zweite bioabbaubare Metall eine elektrische Potenzialdifferenz von mindestens etwa 0,05 V aufweisen; und vorzugsweise im Bereich von etwa 0,05 V bis etwa 3 V, und/oder
b) das erste bioabbaubare Metall Zink, Magnesium oder Eisen umfasst.

6. Transplantatprodukt (20) nach einem der vorstehenden Ansprüche, wobei a) die galvanische Kopplungsstruktur einen elektrischen Strom zwischen der Kathode (24) und der Anode (26) im Bereich von etwa 5 µA bis etwa 500 mA erzeugt, wenn das Transplantat in physiologischer Kochsalzlösung gesättigt ist; und/oder b) die galvanische Kopplung eine Anode (26) und eine Kathode (24) mit einer standardmäßigen Elektrodenpotenzialdifferenz von mindestens etwa 0,05 V aufweist.

7. Transplantatprodukt (20) nach einem der vorstehenden Ansprüche, wobei die Anode (26) und die Kathode (24) dimensioniert und konfiguriert sind, um innerhalb von etwa 180 Tagen nach der Implantation des Gewebetransplantats in einen Patienten abgebaut zu werden.

8. Transplantatprodukt (20) nach einem der vorstehenden Ansprüche, wobei die Kathode (24) ist: a) beabstandet von der Anode (26) um eine Distanz von mindestens 1 mm; oder b) beabstandet um eine Distanz von etwa 1 mm bis etwa 20 mm von der Anode (26).

9. Transplantatprodukt (20) nach einem der vorstehenden Ansprüche, wobei die Kathode (24) eine helixartige Form aufweist und/oder wobei die Anode (26) eine helixartige Form aufweist; wobei wahlweise die Anode und die Kathode beide eine helixartige Form aufweisen und wobei helixartige Windungen der Anode zwischen helixartigen Windungen der Kathode positioniert sind und sich in einer im Allgemeinen parallelen Weise dazu erstrecken.

10. Transplantatprodukt (20) nach einem der vorstehenden Ansprüche, wobei das poröse Transplantatmatrixmaterial bioabbaubar ist und/oder wobei das poröse Transplantatmatrixmaterial bioremodellierbar ist.

11. Transplantatprodukt (20) nach einem der vorstehenden Ansprüche, wobei mindestens eine der Anode (26) und der Kathode (24) durch ein bioabbaubares isolierendes Polymer elektrisch isoliert ist, wobei vorzugsweise sowohl die Anode als auch die Kathode durch ein bioabbaubares isolierendes Polymer elektrisch isoliert sind.

12. Transplantatprodukt (20) nach einem der vorstehenden Ansprüche, umfassend eine Vielzahl der galvanischen Kopplungsstrukturen.

13. Transplantatprodukt (20) nach einem der vorstehenden Ansprüche, wobei die galvanische Kopplung enthält:
(i) eine Kathode (24), umfassend Eisen oder eine Eisenlegierung, und eine Anode (26), umfassend Magnesium oder eine Magnesiumlegierung; oder
(ii) eine Kathode (24), umfassend Eisen oder eine Eisenlegierung, und eine Anode (26), umfassend Zink oder eine Zinklegierung; oder
(iii) eine Kathode (24), umfassend Zink oder eine Zinklegierung, und eine Anode (26), umfassend Magnesium oder eine Magnesiumlegierung.

14. Transplantatprodukt (20) nach einem der vorstehenden Ansprüche, steril verschlossen in medizinischer Verpackung, wobei vorzugsweise die medizinische Verpackung eine feuchtigkeitsbeständige Verpackung ist.

15. Transplantatprodukt (20) nach Anspruch 14, in trockenem Zustand in der medizinischen Verpackung.

## Revendications

1. Produit de greffe électrostimulative à alimentation autonome (20) permettant de traiter un patient, comprenant :
un matériau de matrice de greffe poreux réceptif à la croissance d'un nouveau tissu lorsqu'il est implanté dans le patient ; et
une structure de couple galvanique fixée au matériau de matrice poreux et incluant une cathode (24) constituée d'un premier métal, espacée d'une anode (26) constituée d'un second métal, le premier métal étant différent du second métal, et de préférence, dans lequel le premier métal est un métal biodégradable et le second métal est un métal biodégradable ;
la structure de couple galvanique pouvant être actionnée afin de générer un courant électrique dans un chemin entre l'anode et la cathode, le chemin s'étendant à travers des quantités du matériau de matrice poreux positionné entre l'anode et la cathode.

2. Produit de greffe (20) selon la revendication 1, dans lequel :
le matériau de matrice de greffe poreux est au moins en partie en forme de tube ; et
le chemin s'étend dans une direction axiale le long du tube.

3. Produit de greffe (20) selon la revendication 1 ou 2, comprenant :
une structure stratifiée incluant une pluralité de feuilles (22A, 22B) du matériau de matrice de greffe poreux stratifiées les unes sur les autres ;
dans lequel la cathode (24) est capturée dans la structure stratifiée entre des feuilles adjacentes de ladite pluralité de feuilles, de préférence dans lequel le premier métal est un métal biodégradable ; et
dans lequel l'anode (26) est capturée dans la structure stratifiée entre des feuilles adjacentes de ladite pluralité de feuilles, de préférence dans lequel le second métal est un métal biodégradable.

4. Produit de greffe (20) selon l'une quelconque des revendications précédentes, dans lequel :
a) le matériau de matrice de greffe poreux comprend du collagène ; et/ou
b) dans lequel le matériau de matrice de greffe poreux comprend une ou plusieurs feuilles de tissu membraneux décellularisé, de préférence dans lequel des feuilles de tissu membraneux décellularisé retiennent un ou plusieurs facteurs de croissance natifs d'un tissu source pour les feuilles de tissu membraneux ; et/ou
c) dans lequel le matériau de matrice de greffe poreux comprend du tissu décellularisé sélectionné parmi du tissu sous-muqueux, du tissu dermique, du tissu péricardique, du tissu amniotique, du tissu péritonéal, ou de l'aponévrose.

5. Produit de greffe (20) selon l'une quelconque des revendications précédentes, dans lequel :
a) le premier métal biodégradable et le second métal biodégradable présentent une différence de potentiel électrique d'au moins environ 0,05 V ; et, de préférence, dans la plage comprise entre environ 0,05 V et environ 3 V ; et/ou
b) le premier métal biodégradable comprend du zinc, du magnésium ou du fer.

6. Produit de greffe (20) selon l'une quelconque des revendications précédentes, dans lequel a) la structure de couple galvanique génère un courant électrique entre la cathode (24) et l'anode (26) dans la plage comprise entre environ 5 µA et environ 500 mA lorsque la greffe est saturée en solution saline physiologique ; et/ou b) le couple galvanique présente une anode (26) et une cathode (24) présentant une différence de potentiel d'électrode standard d'au moins environ 0,05 V.

7. Produit de greffe (20) selon l'une quelconque des revendications précédentes, dans lequel l'anode (26) et la cathode (24) sont dimensionnées et configurées afin de se dégrader en environ 180 jours après l'implantation du greffon de tissu dans un patient.

8. Produit de greffe (20) selon l'une quelconque des revendications précédentes, dans lequel la cathode (24) est : a) espacée de l'anode (26) d'une distance d'au moins 1 mm ; ou b) espacée d'une distance comprise entre environ 1 mm et environ 20 mm de l'anode (26).

9. Produit de greffe (20) selon l'une quelconque des revendications précédentes, dans lequel la cathode (24) présente une forme hélicoïdale et/ou dans lequel l'anode (26) présente une forme hélicoïdale ; éventuellement dans lequel l'anode et la cathode présentent toutes deux une forme hélicoïdale, et dans lequel des spires hélicoïdales de l'anode sont positionnées entre et s'étendent d'une manière généralement parallèle aux spires hélicoïdales de la cathode.

10. Produit de greffe (20) selon l'une quelconque des revendications précédentes, dans lequel le matériau de matrice de greffe poreux est biodégradable et/ou dans lequel le matériau de matrice de greffe poreux est bioremodelable.

11. Produit de greffe (20) selon l'une quelconque des revendications précédentes, dans lequel au moins une de l'anode (26) et de la cathode (24) est électriquement isolée par un polymère isolant biodégradable, de préférence dans lequel l'anode et la cathode sont toutes deux électriquement isolées par un polymère isolant biodégradable.

12. Produit de greffe (20) selon l'une quelconque des revendications précédentes, comprenant une pluralité desdites structures de couple galvaniques.

13. Produit de greffe (20) selon l'une quelconque des revendications précédentes, dans lequel le couple galvanique inclut :
(i) une cathode (24) comprenant du fer ou un alliage de fer et une anode (26) comprenant du magnésium ou un alliage de magnésium ; ou
(ii) une cathode (24) comprenant du fer ou un alliage de fer et une anode (26) comprenant du zinc ou un alliage de zinc ; ou
(iii) une cathode (24) comprenant du zinc ou un alliage de zinc et une anode (26) comprenant du magnésium ou un alliage de magnésium.

14. Produit de greffe (20) selon l'une quelconque des revendications précédentes, enfermé de manière stérile dans un emballage médical, de préférence dans lequel l'emballage médical est un emballage résistant à l'humidité.

15. Produit de greffe (20) selon la revendication 14, dans des conditions sèches dans l'emballage médical.
